# EUROPEAN PATENT APPLICATION

(11) **EP 4 574 118 A1**
(43) Date of publication of application: **25.06.2025**
(21) Application number: 23218485.3
(22) Date of filing: 20.12.2023
(51) Int. Cl.: A61F 13/49, A61F 13/491, A61F 13/494, A61F 13/496

(54) **WASHABLE ABSORBENT MALE UNDERWEAR**

(71) Applicant: Essity Hygiene and Health Aktiebolag, 405 03 Göteborg (SE)
(72) Inventor: VASELL, Anna, 405 03 GÖTEBORG (SE); GUSTAFSSON, Anette, 405 03 GÖTEBORG (SE); KARLSON, Tomas, 405 03 GÖTEBORG (SE)
(74) Representative: Nederlandsch Octrooibureau

(57) **Abstract**

Washable absorbent male underwear (1) comprising a textile pant (10) and an absorbent assembly (100), the absorbent assembly (100) comprising
- a liquid permeable layer (120),
- a liquid impermeable layer (150); and

an absorbent structure (140) being arranged in a front portion (11) of the textile pant (10) between the liquid permeable layer (120) and the liquid impermeable layer (150) and comprising a first layer (141) of textile absorbent material, the first layer (141) of textile absorbent material having a first absorbent area; wherein
the first absorbent area defines an area of a first absorbent zone (145) of the absorbent assembly (100), the absorbent assembly (100) further comprising a leak protection zone (155) having an area which is defined by the area of the liquid impermeable layer (150), the absorbent structure (140) completely overlapping the leak protection zone (155), the area of the leak protection zone (155) being greater than the area of the first absorbent zone (145).

## Description

### TECHNICAL FIELD

The present disclosure generally relates to the field of reusable and washable absorbent garments, and more particularly to washable absorbent male underwear comprising a textile pant and an absorbent assembly, the absorbent assembly comprising a liquid permeable layer, a liquid impermeable layer and an absorbent structure being arranged between the liquid permeable layer and the liquid impermeable layer.

### BACKGROUND

Washable and reusable absorbent male underwear as disclosed herein are intended for use by persons suffering from light to moderate urine incontinence.

It is a concern to provide such incontinence garments which as much as possible resemble ordinary underwear and which are comfortable and well-fitting. By necessity, incontinence protection garments include layers and materials which are not present in conventional underwear, such as a liquid permeable top layer which lets liquid pass through into at least one underlying liquid absorbent layer. It is also generally preferred that a liquid barrier layer is arranged on a garment-facing side of the absorbent material to prevent liquid from escaping through the incontinence protection underwear and to wet the user's outer garment. Furthermore, the additional layers are joined to each other and to a pant-material by means of adhesives, stitched seams, etc. which may impart stiffness to parts of underwear for incontinence protection, particularly where multiple joins are arranged on top of each other. The additional layers add bulkiness and stiffness to the underwear and may cause wearer discomfort both as a result of chafing seams and material edges as well as a general unease resulting from a real or perceived conspicuousness of the absorbent underwear. It is therefore a concern to arrange material layers in such a way that the flexibility of the underwear is decreased as little as possible and such that the part of the underwear which is provided for urine absorption is as slim and conformable as possible while offering adequate absorbency and leakage protection.

A particular concern in male underwear, is that it is difficult to optimize absorbent capacity and leakage protection without compromising comfort and conformability of the absorbent male underwear. When constructing incontinence garments for male wearers, the incontinence protection underwear must be constructed taken into consideration the male anatomy, and the shifting location of the micturition point in the male underwear. The position of the male genitalia in the incontinence protection underwear may differ between wearers and may also be different for the same wearer between occasions of use depending on how the wearer has put on the absorbent underwear. Furthermore, the genitalia may shift from an initial position during use of the underwear as a result of the wearer's movements.

An object of the present disclosure is to offer an improved washable absorbent male underwear, having adequate absorbency and leak protection properties for light to moderate urine incontinence as well as a high likeness to ordinary washable male underwear with regard to appearance and comfort.

### SUMMARY

The above and further objects may be achieved with washable absorbent male underwear in accordance with claim 1. Variations of the disclosure are set out in the dependent claims and in the following description.

Disclosed herein is a washable absorbent male underwear comprising a textile pant and an absorbent assembly, the textile pant having an extension in a longitudinal direction y, in a transverse direction x and in a thickness direction z, perpendicular to the longitudinal direction y and the transverse direction x, and comprising a front portion, a rear portion and a crotch portion, the absorbent assembly being permanently secured in the textile pant, the absorbent assembly comprising
- a liquid permeable layer,
- a liquid impermeable layer; and

an absorbent structure being arranged between the liquid permeable layer and the liquid impermeable layer in the front portion of the textile pant and comprising a first layer of textile absorbent material, the first layer of textile absorbent material having a first absorbent area; wherein
the first absorbent area defines an area of a first absorbent zone of the absorbent assembly, the absorbent assembly further comprising a leak protection zone having an area which is defined by the area of the liquid impermeable layer, the absorbent structure completely overlapping the leak protection zone, the area of the leak protection zone being greater than the area of the first absorbent zone.

The absorbent structure completely overlaps the leak protection zone such that the liquid impermeable layer is present everywhere beneath the absorbent structure, i.e., beneath any absorbent layer in the absorbent structure.

In washable absorbent male underwear as disclosed herein, the area of the first absorbent zone may be at most 70% of the area of the leak protection zone.

A technical benefit of this arrangement may include that a relatively bulky liquid acquisition material used in the first layer of textile absorbent material may be arranged in an area of the absorbent assembly where a major part of liquid is expected to insult the washable absorbent male underwear. The liquid acquisition materials contemplated for the washable absorbent male underwear as disclosed herein are generally thick and bulky, and may also be relatively stiff and compression resistant materials, which means that it may be beneficial for reasons of comfort and wearer discretion to minimize the area of the male underwear occupied by such material.

In washable absorbent male underwear as disclosed herein, the leak protection zone may extend in the transverse direction x laterally outside of the first absorbent zone. A portion of the leak protection zone may be arranged at each lateral side of the first absorbent zone in a waist region of the front portion of the absorbent male underwear.

For male incontinence products, the micturition point may vary, due to the male anatomy. Therefore, the risk for urine leakage outside of a typical centrally arranged absorbent core is higher than for a corresponding female incontinence product. It has been found that already at small total amounts of urine in the product, some male products are wet outside of a designated target area, probably due to variation in male micturition point. It has been found that some products are wet in upper parts of the product laterally outside of a centrally arranged absorbent structure, already at lower total amounts of urine in the product in the order of 10 ml or less. It has also been found that the number of products which are wet in the lower part of the product increase with increased total amount of liquid in the product.

Frontal or upper side portions of the absorbent assembly in the washable absorbent underwear as disclosed herein may comprise portions of the liquid impermeable layer and the liquid permeable layer and may optionally include an absorbent layer. Such absorbent material may be provided by the first absorbent layer or by an absorbent layer different from the first absorbent layer or by a combination of the first absorbent layer and an additional layer or layers. The frontal portions of the leak protection zone may comprise absorbent material from a second absorbent layer. The second absorbent layer may extend in the transverse direction x, laterally outside of the first absorbent layer. For reasons of wearer comfort and discretion it may be beneficial that upper side portions of the absorbent assembly contain at most one absorbent layer. The at most one absorbent layer may be a second absorbent layer in which case the upper side portions of the absorbent assembly contain less absorbent layers than the first absorbent area.

The first layer of textile absorbent material and any second and further layer or layers of textile absorbent material may have the same extension in the transverse direction x, implying that the upper side portions of the leak protection zone which extend laterally outside of the first absorbent zone contain only the liquid permeable layer and the liquid impermeable layer.

It has further been found that in washable textile absorbent male underwear which does not include superabsorbent material, the liquid has a higher tendency to transfer downwards under the influence of gravity. This means that the risk for leakage below a superabsorbent free absorbent core placed in a front portion of the incontinence product is higher than in a superabsorbent containing product.

In washable absorbent male underwear as disclosed herein, the leak protection zone may extend in the longitudinal direction y outside of the first absorbent zone and into the crotch portion of the textile pant.

The leak protection zone may extend in the longitudinal direction y outside of the first absorbent zone and through the crotch portion of the textile pant and into the rear portion of the textile pant. A part of the leak protection zone of the absorbent assembly which is arranged in the crotch portion and optionally in the rear portion of the washable male absorbent underwear as disclosed herein may comprise portions of the liquid impermeable layer and the liquid permeable layer and may optionally include an absorbent layer. A technical benefit of arranging a part of the leak protection zone in the crotch portion and optionally in the rear portion of the washable absorbent underwear is that protection against leakage of liquid which runs downward from the original insult point in the front portion of the male absorbent underwear may be avoided or at least reduced. It may be a particular benefit if a lower part of the leak protection zone includes absorbent material which may capture and retain any liquid which reaches the crotch portion and possibly also the rear portion of the male absorbent underwear.

In washable absorbent male underwear as disclosed herein, the leak protection zone may cover at most 70% of the area of the textile pant, such as at most 50% of the area of the textile pant or at most 40% of the area of the textile pant.

In washable absorbent male underwear as disclosed herein, the liquid impermeable layer may be a breathable layer. A technical benefit of limiting the area of the leak protection zone is that parts of the textile pant which are outside the absorbent assembly are left free from liquid impermeable material. Even in washable absorbent male underwear having a breathable liquid impermeable layer, the portions of the pant which are free from liquid impermeable material are generally more breathable and conformable than the parts of the textile pant occupied by the absorbent assembly. This may serve to render the washable absorbent male underwear more like ordinary male underwear. Enhanced breathability may be particularly beneficial in the crotch portion to counteract build-up of perspiration. From a comfort and breathability point of view, the leak protection zone should preferably be as small as possible. However, the size of the leak protection zone is also dependent on the desired degree of leakage protection. In products which are intended for use by persons having only very mild incontinence problems, such as drip incontinence, the leak protection zone may occupy a smaller part of the pant area, e.g., at the front portion of the pant. In products which are intended for reception of greater volumes of liquid, the leak protection zone may occupy a larger part of the pant area and may extend e.g., into the crotch portion of the pant and/or be arranged at the waist portion of the pant. Accordingly, a balance between comfort and leak protection should be considered when deciding the size of the leak protection zone.

In washable absorbent male underwear as disclosed herein, the first absorbent layer may be a spacer fabric layer comprising a top web and a bottom web which are interconnected by pile filaments extending in the thickness direction z between the top web and the bottom web.

A spacer fabric is a relatively non-compressible web material and may retain a large liquid-receiving and liquid containing space within the internal network formed by the pile filaments even when exposed to the compressive forces which normally arise during use of the washable absorbent male underwear.

In washable absorbent male underwear as disclosed herein, the absorbent assembly may be cup-shaped, at least within the first absorbent zone.

In washable absorbent male underwear as disclosed herein, the absorbent assembly may comprise first and second side-panels joined along long curved side-edges by a central join extending in the longitudinal direction from a top edge to a bottom edge of the absorbent assembly, the central join extending perpendicular to the transverse direction x.

Spacer fabrics are relatively stiff and non-extensible materials and may contribute to forming a shape-stable cup-shape or bulge within the area of the absorbent assembly where the spacer fabric is located. A shape-stable cup-shape may have the technical benefit of accommodating the male genitalia, the walls of the cup-shaped absorbent assembly serving to confine the male genitalia and preventing a wearer's penis from moving away from a target absorbent area of the underwear, thus contributing to improve leakage security of the absorbent male underwear.

In washable absorbent male underwear as disclosed herein, the first layer of textile absorbent material may comprise two side panels which are joined by a mid-seam which connects mirror-symmetrical convexly curved side edges of the side panels, the first layer of textile absorbent material being bent along the mid-seam and exhibiting a cup-shape in the thickness direction.

The mid-seam may comprise a number of separate seams, each seam connecting panel parts of different layers in the absorbent assembly.

In washable absorbent male underwear as disclosed herein, the side panels of the first layer of textile absorbent material may be arranged with the convexly curved side edges abutting each other and may be joined by a flatlock stitch.

In washable absorbent male underwear as disclosed herein, the side panels of the first layer of textile absorbent material may be joined by a flatlock stitch in class 600 according to the international standard ISO 4915, such as a flatlock stitch type 607 according to the international standard ISO 4915.

In washable absorbent male underwear as disclosed herein, the absorbent structure may comprise a second layer of textile absorbent material, the first layer of textile absorbent material completely overlapping with the second layer of textile absorbent material.

The second layer of textile absorbent material is applied as an additional absorbent layer between the first absorbent layer and the liquid impermeable layer. The second absorbent layer may be a liquid retaining layer located beneath a liquid acquisition and distribution layer formed by the first absorbent layer. The second absorbent layer may be coextensive with the first absorbent layer, in which case the first and second absorbent layers together form the first absorbent zone. Alternatively, the second absorbent layer may include one or more portions extending outside of the first absorbent layer, thereby creating one or more second absorbent zones. The second absorbent zone or zones contains or contain less absorbent material than the first absorbent zone and is thinner and has less absorbent capacity than the first absorbent zone.

In washable absorbent male underwear as disclosed herein, the second textile absorbent layer only partly overlaps with the first textile absorbent layer, a second absorbent zone being formed by portions of the second textile absorbent layer which are non-overlapping with the first textile absorbent layer. The second absorbent zone or zones thereby comprises or comprise fewer absorbent layers than the first absorbent zone.

In washable absorbent male underwear as disclosed herein, the second textile absorbent layer may be thinner than the first textile absorbent layer.

The provision of a second textile absorbent layer having one or more portions which extend beyond the peripheral edge of the first textile absorbent layer may serve to create a soft, less perceptible, and conspicuous transition at the edges of a relatively stiff and thick first textile absorbent layer. The second absorbent layer may be thinner and more flexible than the first absorbent layer and may provide the absorbent male underwear with absorbent capacity in one or more second absorbent zones arranged outside of the first absorbent zone in the transverse and longitudinal directions. The first absorbent zone may be considered to be a target absorbent area of the absorbent structure in the absorbent assembly and the second absorbent zone or zones may be considered to be back-up absorbent areas which can absorb and retain liquid which either impinges the absorbent assembly outside of the first absorbent zone, or which moves away from the first absorbent zone, e.g., under the influence of gravity.

Although generally less preferred, the second absorbent layer may be a liquid acquisition layer which is arranged on a wearer facing side of a first absorbent layer. The first absorbent layer may then be a liquid receiving and retaining layer also referred to herein as a liquid storage layer. A second absorbent layer being a liquid acquisition layer may have a greater thickness than the first absorbent layer.

In washable absorbent male underwear as disclosed herein, a second absorbent zone may be arranged in the crotch portion of the textile pant.

In washable absorbent male underwear as disclosed herein, the second layer of textile absorbent material may be arranged across a full area of the first layer of textile absorbent material. A technical benefit with the second layer of textile material being arranged to extend over the full area of the first layer of textile absorbent material is that liquid may travel in the thickness direction of the absorbent structure from the first layer of textile absorbent material into the second layer of textile absorbent material over the full area of the first layer of textile absorbent material.

The second textile absorbent layer may be a liquid retaining layer arranged between the first textile absorbent layer and the liquid impermeable layer. Accordingly, liquid which enters the first textile absorbent layer, may be absorbed from the first textile absorbent layer into the second textile absorbent layer and may be retained by the second textile absorbent layer.

In washable absorbent male underwear as disclosed herein, the second textile absorbent layer may be laminated to the liquid impermeable layer. The laminate forms an absorbent liquid impermeable composite which may be handled as a single component when manufacturing the washable absorbent male underwear.

In washable absorbent male underwear as disclosed herein, a second absorbent zone may be arranged at each lateral side of the first absorbent zone in the waist region of the front portion of the textile pant. A technical benefit of arranging second absorbent zones in the waist region is that liquid splashes or drops which impinge the washable absorbent male underwear outside of the first absorbent zone may be captured and retained in the second absorbent zone.

In washable absorbent male underwear as disclosed herein, the layers in the absorbent assembly may be connected with each other by a first construction seam and at least one second construction seam, each construction seam connecting a number of layers being less than the total number of layers in the absorbent assembly, a layer in the first construction seam being the same as a layer in the at least one construction seam and a layer in the first construction seam being different from a layer in the second construction seam.

A technical benefit of such an arrangement may include that no construction seam is made through all layers of the absorbent assembly, such that a stiff compact seam area may be avoided.

By way of example, the first textile absorbent layer may be attached to the second textile absorbent layer by a first construction seam and the second textile absorbent layer may be attached to the liquid permeable layer and to the liquid impermeable layer by a second construction seam, the first and second construction seams being non-overlapping seams.

By providing construction seams involving only some of the layers of the absorbent assembly where at least one layer is shared by the construction seams and at least one layer differs between the construction seams, the absorbent assembly is provided as a coherent unit in which two or more layers of the absorbent assembly are directly connected with each other and at least two layers are only indirectly connected to each other. Construction seams which are separated from each other in this manner may be applied to the absorbent assembly e.g., to provide a less conspicuous edge at the first absorbent layer (spacer material). The divided construction seams, involving fewer layers of material will also be more flexible and conformable than a single seam through multiple layers.

The different material layers in the absorbent assembly are stitched together in zone transitions depending on which materials are present in each zone. The first construction seam may be arranged to connect layers of materials which are present only in the first absorbent zone to ascertain that the first absorbent layer is securely attached in the absorbent assembly. The second construction seam or seams may be seams arranged at edges of the leak protection zone to keep the layers present there together and make it easier to apply a leak protection seal at the edges of the leak protection zone.

In washable absorbent male underwear as disclosed herein, the first construction seam may be a flatlock stitch, such as a flatlock stitch in class 600 according to the international standard ISO 4915, such as a flatlock stitch type 607 according to the international standard ISO 4915.

By *washable* as defined herein is implied an ability of the absorbent male underwear to withstand laundering at 40 °C at least 100 times without any appreciable loss in appearance, integrity and/or functionality. When used herein *laundering* means that the absorbent underwear may be subjected to an aqueous solution containing detergent.

As used herein, the term *absorbent underwear* refers to garments which are worn as a pair of underpants and which are intended to be placed against the skin of the wearer to absorb and contain body fluids such as urine. The underwear as disclosed herein is an undergarment, or underpant intended for use by males suffering from light incontinence, such as drip incontinence, or by males suffering from moderate incontinence. The absorbent underwear is a fabric underwear, also referred to as a textile underwear, and is not a diaper. The underwear according to the present disclosure is intended to be worn in the same manner as conventional underwear and to be laundered after use for reuse as an absorbent incontinent protection underwear.

The term *fabric* as used in the present disclosure may refer to single or multiple layers of textile materials. The fabric may be knitted or woven wherein *knitting* is a method of constructing a fabric by interlocking a series of loops of one or more yarns. There are two major classes of knitting namely warp and weft knitting. *Weaving* is a method or process of interlacing yarns so that they cross each other at right angles to produce woven fabric. The warp yarns run lengthwise in the fabric and the filling threads (weft) or picks, run from side to side.

When used herein a *permanent attachment,* refers to an attachment which is intended to remain intact and not be broken before, during or after use of the washable absorbent male underwear as disclosed herein. A permanently attached absorbent assembly is an absorbent assembly which cannot be separated from the textile pant of the washable absorbent male underwear without breaking or otherwise destroying the washable absorbent male underwear. The absorbent assembly is not necessarily directly bonded to the textile pant, but may instead be attached via leg opening seams or bonded by an intermediate means such as a sealing tape.

As disclosed herein, the absorbent assembly comprises several layers of different materials arranged stacked on each other. The absorbent assembly may comprise the following layers, as seen in a direction from an inner wearer-facing surface of the washable absorbent male underwear towards an outer garment facing surface of the washable absorbent male underwear:
1) A liquid permeable layer. The liquid permeable layer may be any suitable material which can withstand repeated washing. Suitable materials include fibrous knitted or woven materials, such as knitted materials, as well as mesh materials, such as polymer netting, perforated plastic materials, and the like. The layer may comprise or consist of natural fibers such as cotton, regenerated cellulose fibers such as viscose fibers or synthetic fibers such as polyester fibers, polyolefin fibers, etc. The synthetic fibers may be mono-, bi-, or multi-component fibers. Different types of fibers may be combined in the layer, e.g., to promote liquid wicking through the layer while at the same time retaining a dry surface feeling. It may be preferred that the liquid permeable layer comprises at least a portion of inherently wettable (hydrophilic) fibers which may promote liquid uptake and wicking in the layer. The liquid permeable layer may comprise at least a portion of synthetic fibers which do not absorb or retain liquid. However, liquid permeable layers made exclusively of natural or regenerated cellulosic fibers as well as liquid permeable layers made exclusively of synthetic fibers are also contemplated for the washable absorbent male underwear as disclosed herein. Unless having a great open area, a liquid permeable layer made exclusively from synthetic, inherently non-wettable fibers will generally be treated, such as by a surfactant, to render the outer surface of the fibers hydrophilic and wettable.
2) A first absorbent layer. Any suitable knitted, woven or non-woven textile material may be used for the first absorbent layer. The first absorbent layer may be made from synthetic fibers, natural fibers, man-made cellulosic pulp fibers, superabsorbent fibers, or a blend or combination thereof. As disclosed herein, the first absorbent layer used in the washable absorbent male underwear may be a liquid acquisition layer. Suitable liquid acquisition materials are generally bulky materials having a high liquid inlet capacity and a low liquid retention capacity. A non-limiting example of such material is a spacer fabric. The spacer fabric comprises two outer layers, such as two knitted or woven layers which are connected to each other by spacer elements extending generally perpendicular between the layers. The spacer elements are formed from pile filaments which are relatively stiff and resilient as compared to the fibers in the outer layers. The spacer material may be produced by knitting or weaving, as an integral structure. The outer layers may comprise wettable, absorbent fibers, such as natural or regenerated cellulosic fibers and the spacer elements may be made from synthetic material which will retain its resiliency in a wet state. When the washable absorbent male underwear is being used, urine which passes through the top layer and into the first absorbent layer may be absorbed by the outer layers. The non-absorbent pile filaments create a liquid receiving and containing space between the outer layers of the spacer fabric. The liquid containing space may serve as a reservoir for liquid which may be subsequently absorbed by the lower of the two outer layers and optionally of further absorbent material arranged below the first absorbent layer. Liquid may run relatively freely within the space between the outer layers of the spacer material, allowing the liquid to spread out over a greater area of the absorbent structure and increasing the area of the absorbent structure over which liquid transfer to lower layers may take place.
3) Optionally at least one further absorbent layer such as a second absorbent layer. The second absorbent layer may be arranged to receive liquid from the first absorbent layer. The second absorbent layer is preferably made from a high-loft absorbent textile material which can hold liquid in capillaries between the fibers of the material. The second absorbent layer may be a high-loft knitted material. The constituent threads or yams of the second absorbent layer may be knitted in a manner that imparts a relatively lofty and/or dense fibrous network having a high degree of void volume and capillarity making it suitable for absorbing fluid. In some examples, the knit may be a knitted terry cloth, or even a knitted/sheared terry cloth. The constituent threads or yarns may be or may include microfibers, for increased fiber surface area per unit surface area of the second absorbent layer.
4) A liquid impermeable layer. The liquid impermeable layer is typically a polymer film layer. The polymer film is preferably breathable, letting air and water vapor pass through the film but forming a liquid barrier. A breathable polyurethane film may be used for the liquid impermeable layer. The liquid impermeable layer may be directly attached to a lowermost textile layer in the absorbent assembly. The lowermost textile layer may be a second or further absorbent layer in the absorbent assembly. The attachment may be to a liquid retaining absorbent layer or to a thin textile carrier layer. The attachment may be e.g., by a seam or by lamination. Lamination of the liquid impermeable layer to the textile layer may be made by means of a construction adhesive or by extruding the liquid permeable layer onto the textile layer. The construction adhesive may be e.g., a polyurethane based adhesive.

It is to be understood that the absorbent assembly may comprise one or more further absorbent layers in addition to the first absorbent layer and a second absorbent layer, such as one, two, three, four, five or further absorbent layers. The further absorbent layers may have the same or different absorption properties as disclosed herein for the first and second absorbent layers. Any further absorbent layer may be arranged between the first and second absorbent layers. Alternatively, any further absorbent layer may be arranged between the liquid permeable layer and first absorbent layer.

It is to be understood that the first layer of textile absorbent material and any further layer or layers of textile absorbent material may comprise two or more sublayers or strata.

The absorbent assembly may further include a sealing strip. The sealing strip may be adhesively applied to cover a seam connecting first and second panels of a three-dimensionally shaped absorbent assembly. The sealing strip is a liquid impermeable and preferably breathable sealing strip. The sealing strip may be a polymer tape, such as a polyurethane tape.

The textile material in the outer pant is preferably a knitted material such as a cotton knitted material comprising elastic material such as elastane. The washable absorbent male underwear is preferably provided with a separately applied elastic waistband. The elastic waistband may comprise the same knitted material as the outer pant material, or any other suitable textile material. The elastic waistband preferably comprises waist elastic elements, such as elastic threads, or one or more elastic bands.

The liquid permeable layer, the absorbent layer or layers and the liquid impermeable layer of the absorbent assembly may be attached to the outer pant material by means of a seam. A liquid impermeable tape may be applied around the peripheral edges of the absorbent assembly.

### BRIEF DESCRIPTION OF THE DRAWINGS

The washable absorbent male underwear as disclosed herein will be further explained hereinafter by means of non-limiting examples and with reference to the appended drawings wherein:
- Fig. 1: shows a frontal view of a pair of absorbent male underwear seen from the outer, garment-facing side.
- Fig. 2: shows the absorbent male underwear of Fig. 1, when worn by a male user;
- Fig. 3: show a flattened cross section taken along the line III-III through a central part of a front portion of the absorbent male underwear of Fig. 1;
- Fig. 4: shows a perspective view of a spacer material;
- Figs. 5a-5h: show different configurations of absorbent assemblies in absorbent male underwear as disclosed herein;
- Figs. 6a-6c: show exploded cross sectional views of the layers in three different absorbent assemblies for use in absorbent male underwear as disclosed herein; and
- Figs. 7a-7b: show cross sectional views of the absorbent assemblies in Figs. 6a-6c, with the position of constructional seams indicated.

### DETAILED DESCRIPTION

It is to be understood that the drawings are schematic and that individual components or features, such as layers of material are not necessarily drawn to scale. The absorbent male underwear shown in the figures is provided as an example only and should not be considered limiting to the invention as disclosed herein.

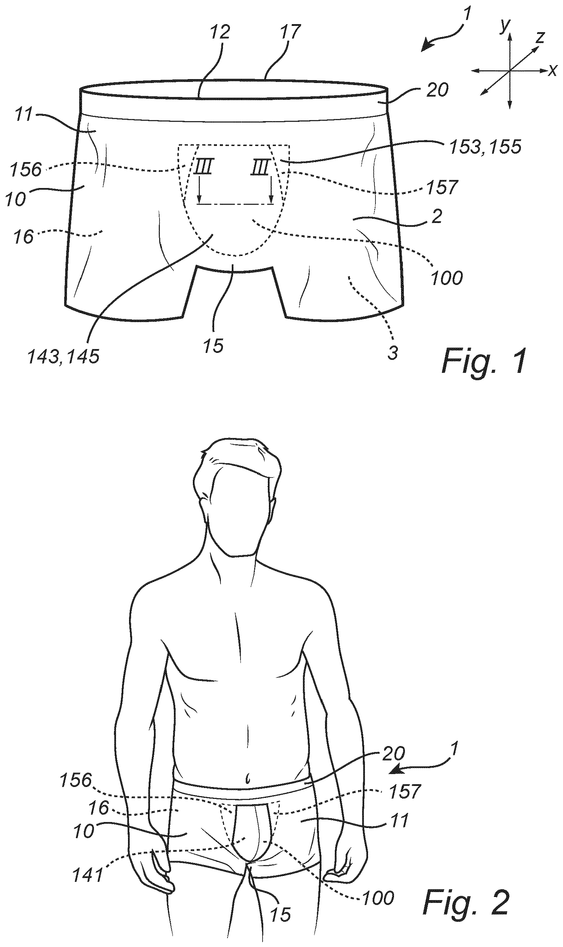

The washable absorbent male underwear disclosed herein should not be construed as being limited to the aspects set forth herein but can be varied within the scope of the appended claims. Although the washable absorbent male underwear shown in Figs. 1 and 2 takes the form of boxer style pants, it is to be understood that the washable absorbent male underwear may have any useful type of male underwear as known in the art.

Fig. 1 shows a frontal view of absorbent male underwear 1 as seen from the outer, garment-facing side 2. Fig. 2 shows the absorbent male underwear 1 of Fig. 1 when worn by a male user. The absorbent male underwear comprises a textile pant 10 and includes an absorbent assembly 100 which is permanently secured in the textile pant 10.

As shown in Fig. 1, the textile pant 10, the whole absorbent male underwear 1 and the absorbent assembly 100 have an extension in a longitudinal direction y, in a transverse direction x and in a thickness direction z which is perpendicular to the longitudinal direction and the transverse direction. The absorbent male underwear 1 has a front portion 11, a rear portion 16 and a crotch portion 15 bridging the front and rear portions 11,16. The male underwear 1 which is shown in Figs. 1 and 2 includes an elastic waistband 20 for providing improved retention of the absorbent male underwear 1. An elastic waistband is an optional component of the absorbent male underwear 1 as disclosed herein and may be applied as a separate component to the textile pant 10 of the absorbent male underwear 1 or may be integrally formed with the textile pant 10, e.g., as a portion of the textile pant 10 having increased elasticity as compared to lower parts of the textile pant 10.

The material of the textile pant 10 may be any suitable fabric, including fabrics constructed from natural fibers, regenerated cellulose fibers, synthetic fibers and mixtures and combinations thereof. Useful natural fibers include cotton, wool, silk, bamboo, hemp, flax, ramie, coir, or banana. Useful regenerated cellulose fibers include rayon, viscose, modal, lyocell and Tencel. Useful synthetic fibers include polyamide, acrylic, polyester.

As set out herein, the textile material may comprise a blend or a combination of any of the abovementioned natural fibers, regenerated cellulosic fibers and synthetic fibers. The fibers may be recycled fibers. The textile material may comprise elastically stretchable material, e.g., elastane filaments, so that the absorbent male underwear 1 can conform to the wearer's body and adapt to the wearer's movements. The elastic conformability of the textile pant contributes to preventing liquid from leaking out through the leg openings and to keep the absorbent assembly 100 in place against the wearer's body. A textile material is generally breathable, allowing vapor to escape from the wearer's skin and from the absorbent assembly 100. Advantageously, the material in the textile pant 10 is a combination of cotton and elastane.

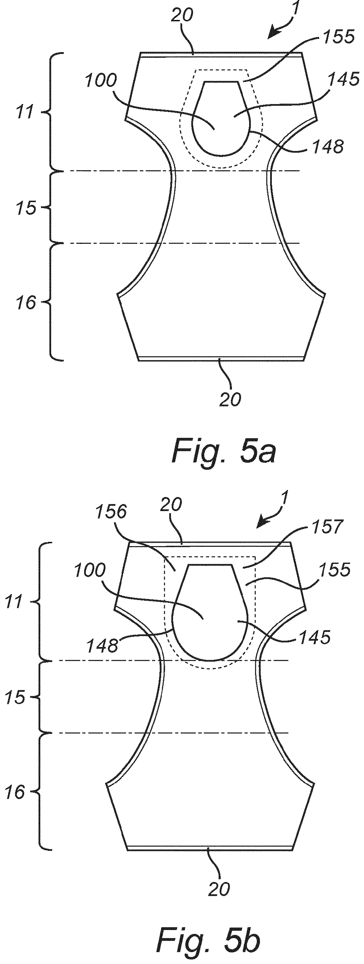

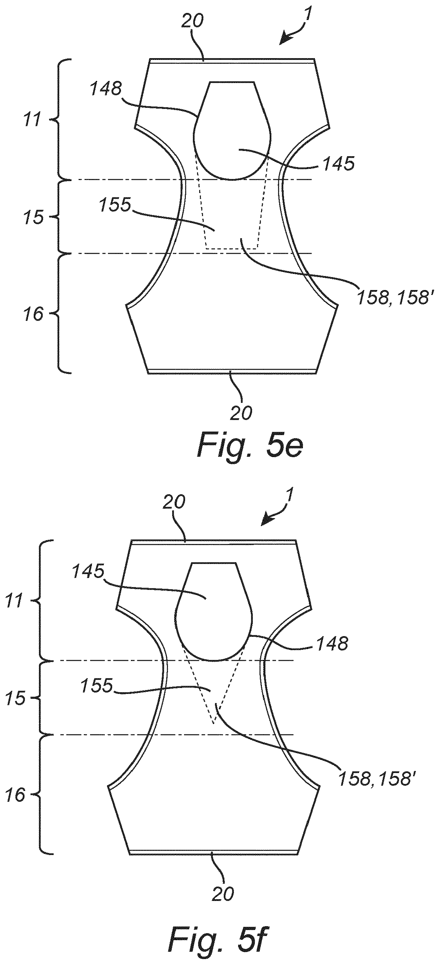

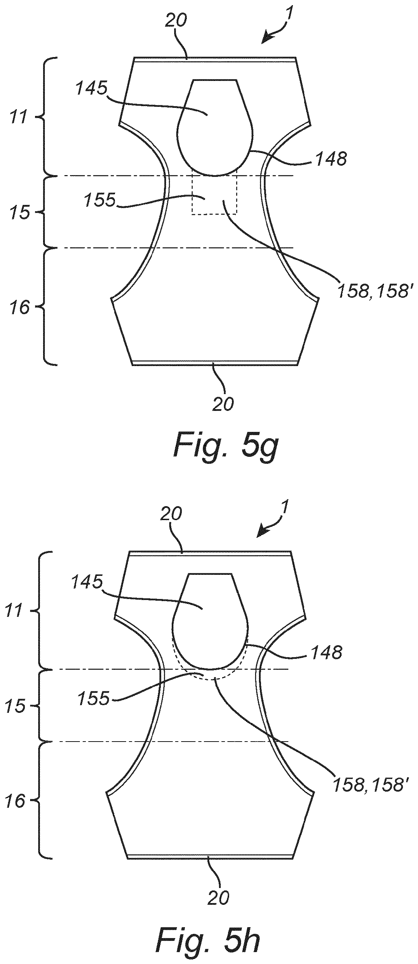

The absorbent assembly 100 is integrated with the textile pant 10 and is positioned in a region of the textile pant 10 mostly likely to be exposed to male incontinence to provide absorbent capacity and leakage protection. In the absorbent male underwear 1 shown in Figs. 1 and 2, the absorbent assembly is located in an area of the front portion 11 of the absorbent male underwear 1 extending in the longitudinal direction y from a waist edge 12 towards the crotch portion 15. With further reference to the example shown in Figs. 1 and 2, the absorbent assembly 100 has a generally triangular shape with a rounded lower corner positioned at the crotch portion 15 of the absorbent male underwear 1. However, as disclosed herein, the absorbent assembly may extend downward into the crotch portion 15 of the absorbent male underwear 1 and may even extend through the crotch portion 15 and into the rear portion 16 of the absorbent male underwear. It is to be understood that the absorbent assembly 100 may take any form within the scope of protection of the claims, such as the forms shown in Figs. 5a-5h.

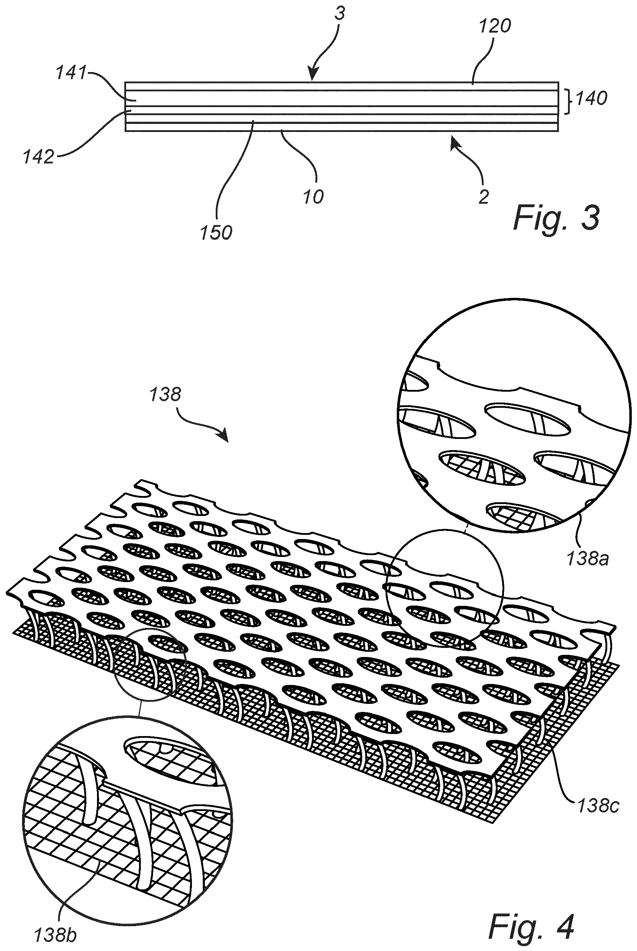

As disclosed herein, the absorbent assembly 100 comprises several layers of different materials arranged stacked on each other to form a reliable absorbent structure capable of quickly absorbing and storing discharges resulting from urinary incontinence. With reference to Fig. 3, the absorbent assembly 100 comprises a liquid permeable layer 120, a liquid impermeable layer 150 and an absorbent structure 140 arranged between the liquid permeable layer 120 and the liquid impermeable layer 150 and being positioned in the front portion 11 of the textile pant 10. The absorbent structure 140 comprises a first layer 141 of textile absorbent material and may optionally comprise at least a second layer 142 of textile absorbent material. It is to be understood that the first layer 141 of textile absorbent material and any further layer or layers of textile absorbent material may comprise two or more sublayers or strata.

As shown in Fig. 1, the first layer of textile absorbent material 141 has a first absorbent area 143 which defines an area of a first absorbent zone 145 of the absorbent assembly 100.The absorbent assembly further has a leak protection zone 155 having an area 153 which is defined by the area of the liquid impermeable layer 150 and which is greater than the area of the first absorbent zone 145. The absorbent structure 140 completely overlaps the leak protection zone 155, which means that the liquid impermeable layer 150 is present everywhere between the absorbent structure 140 and the textile pant 10.

In the absorbent male underwear 1 which is shown in Figs. 1 and 2, the leak protection zone 155 extends in the transverse direction x laterally outside of the first absorbent zone 145 in an upper portion of the absorbent assembly 100 located below the elastic waistband 20. Accordingly, a portion 156, 157 of the leak protection zone 155 is arranged at each lateral side of the first absorbent zone 145 in the waist region of the front portion 11 of the textile pant. 10. The portions 156, 157 of the leak protection zone 155 which are located laterally outside of the first absorbent zone 145 form triangular leak protection "ears" 156, 157 along longitudinally extending side edges of the first absorbent zone 145.

The first absorbent zone 145 may also be referred to as a liquid storage zone which is intended to absorb and retain liquid in the absorbent male underwear 1. Ideally, all liquid which is emitted to the absorbent male underwear 1, should enter the absorbent assembly 100 in the first absorbent zone 145 to allow the liquid to be captured and stored in the first layer 141 of textile absorbent material. However, it has been found that for incontinent male persons, the micturition point in the absorbent male underwear 1 may vary not only between users but also for a same incontinent person depending on how the user's penis happens to be positioned in the absorbent male underwear 1. Studies were made using TENAO Men Level 2 disposable incontinence protectors from Essity Hygiene and Health AB. The TENAO Men Level 2 disposable incontinence protectors are incontinence protectors having a shape similar to that of the absorbent assembly 100 in Figs. 1 and 2 and which are used while being releasably attached in a pair of ordinary male underpants in a position similar to that of the absorbent assembly 100 in Figs. 1 and 2. The studies revealed that up to 10% of the products were wet in the upper outer corners already at a total amount of urine in the product of 10 ml or less.

If the absorbent structure does not cover portions of the underwear corresponding to the upper outer corners of the TENAO Men Level 2 disposable incontinence protector, there is a non-negligible risk of leakage from such products. As set out herein, it is a concern when making absorbent male underwear for active male incontinent persons leading a normal social life that the garment is comfortable and discrete and as much like ordinary underwear as possible. Accordingly, there is a constant conflict between the amount of absorbent material needed to obtain high leakage security and the desire for a non-bulky and comfortable pant which can be inconspicuously worn beneath conventional clothing. With the absorbent male underwear 1 in Figs. 1 and 2, the risk for leakage in an upper part of the product has been considerably reduced by the provision of the leak protection ears 156, 157. In the example shown in Figs. 1 and 2, the leak protection ears 156, 157 contain the liquid impermeable layer 150, the liquid permeable layer 120 and optionally an absorbent layer such as a second layer 142 of textile absorbent material. In Figs. 1 and 2, the first layer 141 of textile absorbent material is not present in the leak protection ears 156, 157. However, it is to be understood that the leak protection ears 156, 157 may contain the liquid impermeable layer 150, the liquid permeable layer 120 and optionally one of either the first layer 141 of textile absorbent material and the second layer 142 of textile absorbent material. The leak protection ears 156, 157 may comprise fewer material layers than the first absorbent zone 145. Thus, the leak protection ears 156, 157 are thinner and more flexible portions of the absorbent assembly than the first absorbent zone 145. Any small amount of liquid which may hit the absorbent assembly 100 within the area of the leak protection ears 156, 157 is captured in the liquid permeable layer 120 and in any optional layer of textile absorbent material and is prevented by the liquid impermeable layer 150 from penetrating out from the absorbent assembly and wetting the textile pant 10 and ultimately a wearer's outer clothing.

The absorbent assembly 100 of the absorbent male underwear as disclosed herein may be a three-dimensionally shaped absorbent assembly 100 as best illustrated by Fig. 2. A three-dimensionally shaped absorbent assembly 100 provides a cup-shaped containment space for male genitalia. A pre-formed cup-shape may accommodate the male genitalia and the walls of the cup-shaped containment space may serve to confine the male genitalia to a predetermined portion of the absorbent male underwear 1 by preventing a wearer's penis from moving away from a target absorbent area of the underwear. A three-dimensionally shaped absorbent assembly 100 may, thus, contribute to improve leakage security of the absorbent male underwear.

A three-dimensional cup-shape may be formed by providing two substantially mirror-symmetrically shaped side-panels with convexly curved edges from each layer of the absorbent assembly 100 and stitching the edges together along a central join.

It is well known to skilled workers in many arts, including undergarment manufacture, that flexible sheet materials will deform and form three-dimensional shapes when curved edges are forced to come together by attachment means. As the pieces are stitched together, they bend outwards in the thickness direction z along the curved central join and provide a physical space on the inside of the absorbent male underwear 1. Accordingly, a cup-shaped space may be formed along the lower ventral abdomen of the user for accommodating the genitals in a comfortable way.

Side panels of the first layer 141 of textile absorbent material may be arranged with the convexly curved side edges abutting each other and being joined by a flatlock stitch. The flatlock stitch may be a flatlock stitch in class 600 according to the international standard ISO 4915, such as a flatlock stitch type 607 according to the international standard ISO 4915.

The international standard ISO 4915 classifies, designates, describes and illustrates various kinds of stitch types. Stitch types are divided into six classes (100-600) and advantageously chain stitches in class 600 may be used for joining the first and second side-panels of the first absorbent layer 141 described herein. Preferably the stitch is chosen from one of stitch types 602, 603, 604, 605, 606, 607, 608 or 609 in the 600 series.

A further way of creating a three-dimensionally shaped absorbent assembly 100 may be by press-forming an absorbent assembly containing at least one thermoformable layer.

The fluid distribution studies made using TENA^{®} Men Level 2 disposable incontinence protectors further revealed that with increasing amounts of liquid in the product, the liquid travels downward in the product under the influence of gravity. The TENA^{®} Men Level 2 disposable incontinence protectors is a disposable, non-washable product containing superabsorbent polymers which may chemically bind liquid and retain the liquid in the incontinence protector. However, in washable absorbent male underwear as disclosed herein and being free from superabsorbent material, the liquid may continue to flow downwards in the product and eventually cause leakage in the crotch portion of the absorbent male underwear 1.

As set out herein, the leak protection zone 155 may extend in the longitudinal direction y outside of the first absorbent zone 145 and into the crotch portion 15 of the textile pant 10.

It is further conceivable that the leak protection zone 155 extends in the longitudinal direction y outside of the first absorbent zone 145, through the crotch portion 15 of the textile pant 10 and into the rear portion 16 of the textile pant.

The area of the first absorbent zone 145 may be less than the area of the leak protection zone, such as at most 70% of the area of the leak protection zone 155. Due to a difference in number of material layers in the first absorbent zone 145 and in parts of the absorbent assembly 100 which are located outside the periphery of the first absorbent zone 145, the thickness and rigidity of the absorbent assembly is generally higher within the area of the first absorbent zone 145 than in the parts of the absorbent assembly 100 which are located outside the first absorbent zone 100. For reasons of comfort and wearer discretion it may be beneficial to minimize the area of the male underwear which is thickened by an absorbent material layer or layers.

It may also be preferred that the leak protection zone covers at most 70% of the area of the textile pant, such as at most 50% of the area of the textile pant or at most 40% of the area of the textile pant.

Parts of the textile pant which are outside the absorbent assembly are thereby left free from liquid impermeable material. Even in washable absorbent male underwear having a breathable liquid impermeable layer, the portions of the pant which are free from liquid impermeable material are generally more breathable and conformable than the parts of the textile pant occupied by the absorbent assembly.

As set out herein, the absorbent assembly 100 comprises at least the following layers, as seen in a direction from an inner wearer-facing surface 3 of the absorbent male underwear 1 towards an outer garment-facing surface 2 of the absorbent male underwear 1:
The liquid permeable layer 120 is arranged closest to the inner wearer-facing surface 3 of the absorbent male underwear 1 when the absorbent assembly 100 is integrated in the textile pant 10. The liquid permeable layer 120 may be any suitable material which can withstand repeated washing. Examples of suitable materials include fibrous knitted or woven materials, such as knitted materials, as well as mesh materials, such as polymer netting, perforated plastic materials, and the like. The liquid permeable layer 120 may comprise or consist of natural fibers such as cotton, regenerated cellulose fibers such as viscose fibers or synthetic fibers such as polyester fibers, polyolefin fibers, e.g., polypropylene fibers, etc. The synthetic fibers may be mono-, bi-, or multi-component fibers.

Different types of fibers may be combined in the liquid permeable layer 120, e.g., to promote liquid wicking through the layer while at the same time retain a dry surface in contact with a wearer's skin. It may be preferred that the liquid permeable layer 120 comprises at least a portion of inherently wettable (hydrophilic) fibers which may promote liquid uptake and wicking in the layer. It may also be preferred that the liquid permeable layer 120 comprises at least a portion of synthetic fibers which do not absorb or retain liquid.

The liquid permeable layer 120 may, for example, comprise or consist of a combination of viscose or other regenerated fibers and polyolefin fibers, such as polypropylene fibers. However, liquid permeable layers made exclusively of natural or regenerated cellulosic fibers as well as liquid permeable layers made exclusively of synthetic fibers are also contemplated for the washable absorbent male underwear 1 as disclosed herein.

The liquid permeable layer 120 may comprise from 20% to 100% of synthetic fibers, more preferably from 40% to 100% of synthetic fibers and most preferably from 60% to 100% of synthetic fibers. The skin-facing top layer is a water-permeable material layer allowing body fluids to penetrate the layer and transfer into underlying absorbent layer or layers.

The liquid permeable layer 120 may have openings formed therein. Unless having a great open area, a liquid permeable layer 120 made exclusively from synthetic, inherently non-wettable fibers will generally be treated, such as by a surfactant, to render the outer surface of the fibers hydrophilic and wettable.

The absorbent structure 140 comprises at least a first layer 141 of textile absorbent material. The first absorbent layer is a material which can act as a liquid acquisition layer and which may serve to distribute liquid in the absorbent structure and optionally transfer liquid to an underlying second absorbent layer, such as a liquid storage layer. The first layer 141 of textile absorbent material may be arranged in direct contact with the liquid permeable layer 120 to enable the moisture to quickly enter the first layer 141 of textile absorbent material and to spread, e.g., by wicking in the first absorbent layer such that liquid is moved away from the wearer and optionally transferred into a second layer 142 of textile absorbent material arranged underneath the first layer 141 of textile absorbent material. Good fluid distribution properties in the first layer 141 of textile absorbent material enables efficient spread of liquid and allows liquid to be received over a greater area of an underlying second layer 142 of textile absorbent material. This property is particularly beneficial for users suffering from stress incontinence as spurts of urine may cause the first layer 141 of textile absorbent material to be rapidly saturated at the point of impact. By distributing the volume of the liquid over a wider receiving area in the first layer 141 of textile absorbent material, the overall liquid uptake capacity of the first layer 141 of textile absorbent material may be increased.

Advantageously, the first layer 141 of textile absorbent material is a spacer fabric 138 comprising a top layer 138a and a bottom layer 138b and an interconnecting layer 138c of pile filaments between the top layer 138a and the bottom layer 138b as seen in Fig. 4. The top layer 138a and the bottom layer 138b of the spacer fabric 138 may have different configurations to control the flow of the liquid in the spacer fabric 130 and liquid transfer to an optional lower absorption layer.

A spacer fabric 138 is more compression resistant than other components that may be used for fluid flow control in absorbent articles but at the same time has a relatively low density. This allows for a slim and efficient fluid flow control member that will maintain its structure and fluid flow control properties when pressure is exerted on it during use of the absorbent male underwear 1.

The bottom layer of the spacer fabric may have a denser structure with smaller openings than the top layer. The pile filaments 138c may together form channels, preferably in a diagonal direction of the fabric, creating an internal channel network promoting efficient distribution of liquid in the first layer 141 of textile absorbent material. The spacer fabric 138 is a highly porous material in which a free volume is present, also when the absorbent structure 140 is exposed to pressure exerted by a user wearing the absorbent male underwear 1, as a result of high resistance to compression. Due to the free volume, the spacer fabric 138 can receive and temporarily hold a relatively large liquid volume. Thus, body liquids discharged within the first absorbent zone 145 of the absorbent assembly 100 can be effectively received into to the spacer fabric 138 and can flow in the internal channel network in the spacer fabric 138 to be distributed over the area of the first absorbent zone 145. If a second layer 142 of textile absorbent material is present underneath the first layer 141 of textile absorbent material, the distributed liquid can be transferred to the second layer 142 of textile absorbent material over an area which may be as large as the first absorbent zone 145. The liquid which is transferred to the optional second absorbent layer may be absorbed and retained in that layer. The first absorbent layer and the second absorbent layer may be considered to form a liquid storage zone in the absorbent male underwear 1, the liquid storage zone having the same extension in the plane as the first absorbent zone 145 which is defined by the area of the first layer 141 of textile absorbent material.

The top layer 138a of the spacer fabric 138 may have an open structure to ensure rapid inflow and effective distribution of liquid in the spacer fabric 138. The spacer fabric 138 is preferably substantially free from absorbing fibres and does not contain superabsorbent material.

The spacer fabric 138 preferably comprises thermoplastic polymer fibres, preferably selected from but not limited to, polyesters, polyamides and polyolefins such as polyethylenes and polypropylenes, and may be a mixture or combination of any of these. The spacer fabric 138 may also advantageously comprise fibres from cotton, viscose and/or modal. For a preferred spacer fabric 138, the yarns of the first and second layers 138a, 138b as well as the pile filaments 138c are of polyester.

The spacer fabric 138 may also contain surfactant to facilitate liquid penetration into the spacer fabric 138. In absorbent structures 140 comprising a second layer 142 of textile absorbent material it may also be desirable that the spacer fabric 138 can be quickly drained from liquid, thus maintaining free volume capacity for a next gush of urine.

The spacer fabric 138 is advantageously orientated in the absorbent front panel 100 such that the top layer 138a of the spacer fabric 138 is facing and preferably is in contact with the liquid permeable layer 120, and the bottom layer 138b of the spacer fabric 138 is facing the liquid impermeable layer 1.

If present, the second layer 142 of textile absorbent material is arranged to receive liquid from the first absorbent layer 130, e.g., from a spacer layer. The second layer 142 of textile absorbent material is preferably made from a high-loft absorbent textile material serving as a liquid absorbing and storage layer which can hold liquid in capillaries between the fibers of the material.

Advantageously the second layer 142 of textile absorbent material comprises materials such as commercially processed cotton, modal, lyocell or combinations thereof. Also, one or more of polyester, polyamide and/or combinations thereof may be preferred in fiber components of yarn or threads of fabrics for the second layer 142 of textile absorbent material.

The constituent threads or yams of the second layer 142 of textile absorbent material may be knitted or woven in a manner that imparts a relatively lofty and/or dense fibrous network having a high degree of void volume and capillarity making it suitable for absorbing and storing fluid. In some examples, the knit may be a knitted terry cloth, or even a knitted/sheared terry cloth. The constituent threads or yarns may be or may include microfibers, for increased fiber surface area per unit surface area of the second layer 142 of textile absorbent material.

The absorbent structure 140 may comprise one or more further absorbent layers in addition to the first layer 141 of textile absorbent material. It may be preferred that the absorbent structure comprises at least a second layer 142 of textile absorbent material which may provide liquid retention and storage properties to the absorbent structure 140 as set out herein. Further absorbent layers may have the same or different absorption properties as the first and optional second layers 141, 142 of textile absorbent material. Such further absorption layers may add absorption capacity and/or other absorption properties such as liquid distribution properties, i.e., fluid handling properties. The further absorbent layer(s) may be arranged between the first and second layers 141, 142 of absorbent material. Alternatively, a further absorbent layer may be arranged between the liquid permeable layer 120 and the first layer 141 of textile absorbent material.

A liquid impermeable layer 150 is included as a barrier layer in the absorbent assembly 100 to prevent absorbed urine from passing from the absorbent structure 140 to the textile pant 10 and to outer clothing. The liquid impermeable layer 150 is typically a polymer film layer. The polymer film is preferably breathable, letting air and water vapor pass through the film yet forming a barrier against liquid penetration. The liquid impermeable layer 150 may comprise a film formed in whole or in part of a polyurethane or polyester based resin. Advantageously, a breathable polyurethane film may be used for the liquid impermeable layer 150. The polymer film may be formed by extrusion or other application of molten thermoplastic resin directly onto an outward-facing surface of the absorbent structure 140, such as a second layer 142 of textile absorbent material, to form an absorbent-liquid impermeable laminate. Alternatively, the liquid impermeable layer 150 is laminated to the absorbent structure 140, such as by adhesive. The adhesive may be a polyurethane based adhesive. In a further example a second layer 142 of textile absorbent material and the liquid impermeable layer 150 form separate layers in the absorbent assembly 100.

For the purpose of preventing growth of microorganisms promoted by absorbed urine, and counteracting mal odors, one or more anti-microbial agents may be included in or among the materials present in the absorbent assembly 100 and/or in any other component of the absorbent male underwear 1

With reference now to Figs. 5a-5h, there is shown a number of different configurations of absorbent assemblies 100 in absorbent male underwear 1 as disclosed herein. The absorbent assemblies 100 are shown positioned on the inner surface of absorbent male underwear 1, with the first absorbent zone 145 located centrally on the front portion 11 of the absorbent male underwear 1. The absorbent male underwear 1 is a brief-style underwear which is shown with non-connected side seams and in a flat-out state.

In Figs. 5a and 5b, the leak protection zone 155 extends outside the edges of the first absorbent zone 145 along a full periphery 148 of the first absorbent zone 145, implying that additional leakage security is provided above the first absorbent zone 145, below the first absorbent zone 144 and along the longitudinal side edges of the first absorbent zone 145. In Fig. 5a, the extension of the leak protection zone 155 outside the peripheral edges of the first absorbent zone 145 is substantially the same at all points on the periphery 148 of the first absorbent zone 145.

In Fig. 5b, the arrangement of the leak protection zone 155 is similar to that in Fig. 5a but differs in that the leak protection zone 155 has a widened portion 156, 157 of the leak protection zone 155 arranged at each lateral side of the first absorbent zone 145 in the waist region of the front portion 11 of the absorbent male underwear 1.

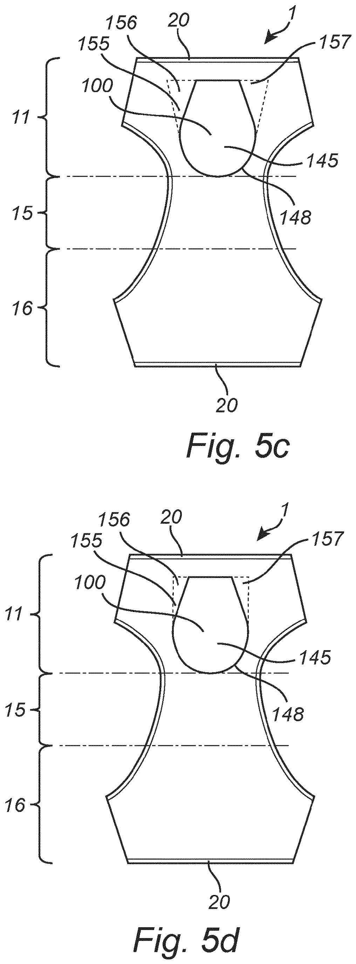

The absorbent male underwear 1 shown in Figs. 5c and 5d have a configuration of the absorbent assembly 100 which is similar to that which has already been described herein in connection with Figs. 1 and 2, i.e., with a portion 156, 157 of the leak protection zone 155 being arranged at each lateral side of the first absorbent zone 145 in the waist region of the front portion 11 of the absorbent male underwear 1. The difference between the configurations in Figs. 5c and 5d is that the side portions 156, 157 of the leak protection zone 155 are somewhat wider in Fig. 5c than in Fig. 5d.

The absorbent male underwear 1 shown in Figs. 5e-5h, all have a portion 158 of the leak protection zone 155 extending downward from the periphery 148 of the first absorbent zone 145 into the crotch portion 15 of the absorbent male underwear 1. As set out herein, the purpose of such arrangement is to capture any liquid running under the influence of gravity from the first absorbent zone 145 downwards into the crotch portion 15 of the absorbent male underwear 1. It may be preferred that the portion 158 of the leak protection zone 155 which is located in the crotch portion 15 of the absorbent male underwear contains a second layer 142 of textile absorbent material thereby creating a second absorbent zone 158' in the crotch portion 15 of the washable absorbent male underwear 1 as disclosed herein. It is to be understood that the configurations of the leak protection zones 155 shown in Figs. Figs. 5a-5d can be freely combined with any one of the configurations shown in Figs. 5e-5h to provide additional leakage security both at an upper part of the absorbent male underwear 1 and at a lower part of the absorbent male underwear 1.

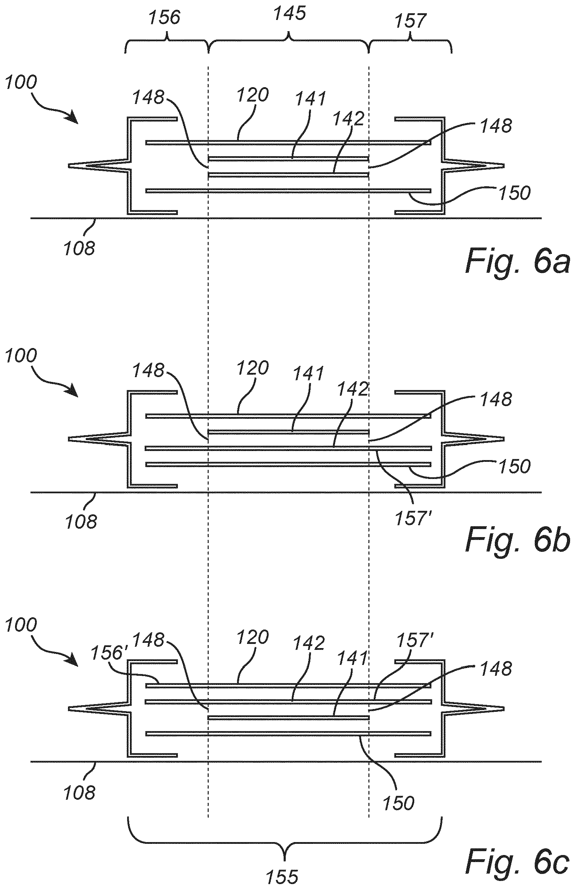

Figs. 6a-6c are cross sectional views of different arrangements of a first and a second layer 141, 142 of textile absorbent material in an absorbent assembly 100 of absorbent male underwear 1 as disclosed herein. All the absorbent assemblies in Figs. 6a-6c comprise a first layer 141 of textile absorbent material and a second layer 142 of textile absorbent material. The layered materials are joined by a folded-over sealing strip 108 which is around the perimeter of the layered stack of materials forming the absorbent assembly 100 to be integrated into the textile pant 10.

Advantageously a first edge portion of the folded-over sealing strip 108 is applied and adhered to the outer surface of the liquid permeable layer 120 along the entire peripheral edge of the liquid permeable layer 120. Thereafter the folded-over sealing strip 108 is folded around the edge of the stack of layered material such that a middle section of the barrier strip sticks to itself and a second edge portion of the folded-over sealing strip 108 is adhered to the outer surface of the liquid impermeable layer 150 along the entire peripheral edge on the garment-facing side of the liquid impermeable layer 150. Thereby a liquid tight seal is formed around the outer edge of the absorbent assembly 100, preventing any liquid leaking from the absorbent assembly 100.

The absorbent assembly 100 is attached to the wearer-facing side of the textile pant 10 centered on a longitudinally extending midline of the front portion 11. The absorbent assembly 100 may be stitched to the textile pant 10 with a straight stitch which is arranged around the periphery of the absorbent assembly 100. The straight stitch is preferably formed in a part of the folded-over sealing strip 108 which is arranged outside the peripheral edge of the stack of layers of the absorbent assembly 100. By arranging the seam at a distance from the edge of the stack of layers in the absorbent assembly 100, side leakage from the absorbent assembly 100 is prevented. Thus, the absorbent assembly 100 is provided as an integrated part of a complete washable absorbent male underwear and is not intended to be removable or replaceable.

In Fig. 6a, the first and second layers 141, 142 of textile absorbent material have the same extension in the transverse direction x, implying that the portions 156, 157 of the leak protection zone 155 which extend laterally outside of the first absorbent zone 145 contain only the liquid permeable layer 120 and the liquid impermeable layer 150.

In Fig. 6b, the first layer 141 of textile absorbent material is arranged between the second layer 142 of textile absorbent material and the liquid permeable layer 120 and has a smaller extension in the transverse direction x than the second layer 142 of textile absorbent material which has the same extension in the transverse direction as the liquid permeable layer 120 and the liquid impermeable layer, implying that the portions 156, 157 of the leak protection zone 155 which extend laterally outside of the first absorbent zone 145 contain the liquid permeable layer 120, the liquid impermeable layer 150 and the second layer 142 of textile absorbent material, thereby forming second absorbent zones 156', 157' outside the periphery 148 of the first absorbent zone 145. In the Fig. 6b arrangement, the first layer 141 of textile absorbent material may be a liquid acquisition layer, such as a liquid acquisition layer comprising a spacer material as disclosed herein.

In Fig. 6c, the first layer 141 of textile absorbent material is arranged between the second layer 142 of textile absorbent material and the liquid impermeable layer 150 and has a smaller extension in the transverse direction x than the second layer 142 of textile absorbent material which has the same extension in the transverse direction as the liquid permeable layer 120 and the liquid impermeable layer, implying that the portions 156, 157 of the leak protection zone 155 which extend laterally outside of the first absorbent zone 145 contain the liquid permeable layer 120, the liquid impermeable layer 150 and the second layer 142 of textile absorbent material, thereby forming second absorbent zones 156', 157' outside the periphery 148 of the first absorbent zone 145.

In the Fig. 6c arrangement, the second layer 142 of textile absorbent material is an upper layer and may be a liquid acquisition layer, such as a liquid acquisition layer comprising a spacer material as disclosed herein. The first layer 141 of textile absorbent material may be a liquid receiving and retaining layer, also referred to herein as a liquid storage layer. A second layer 142 of textile absorbent material being a liquid acquisition layer may have a greater thickness than the first layer 141 of textile absorbent material.

As set out herein the first absorbent zone 145 may also be referred to as a liquid storage zone. The liquid storage zone will always contain the first layer 141 of textile absorbent material and may optionally contain at least a second layer 142 of textile absorbent material.

As set out herein, the provision of a second layer 142 of textile absorbent material having one or more portions which extend beyond the peripheral edge of the first layer 141 of textile absorbent material may serve to create a soft, less perceptible, and conspicuous transition at the periphery 148 of the first absorbent zone 148. In absorbent assemblies 100, such as the absorbent assembly in Figs. 6a and 6b, where the first layer 141 of textile absorbent material is an upper layer which may typically be a relatively stiff and thick liquid acquisition material such as a spacer material, the edge masking effect of the second layer 142 of textile absorbent material may be particularly beneficial. In such case, the second absorbent layer is preferably thinner and more flexible than the first absorbent layer and may provide the absorbent male underwear with absorbent capacity in one or more second absorbent zones 156, 157 arranged outside of the first absorbent zone in the transverse and longitudinal directions x, y. The first absorbent zone 145 is a target absorbent area of the absorbent structure 140 in the absorbent assembly 100 and the second absorbent zone 156, 157 or zones may be considered to be back-up absorbent areas which can absorb and retain liquid which either impinges the absorbent assembly outside of the first absorbent zone 145, or which moves away from the first absorbent zone 145, e.g., under the influence of gravity.

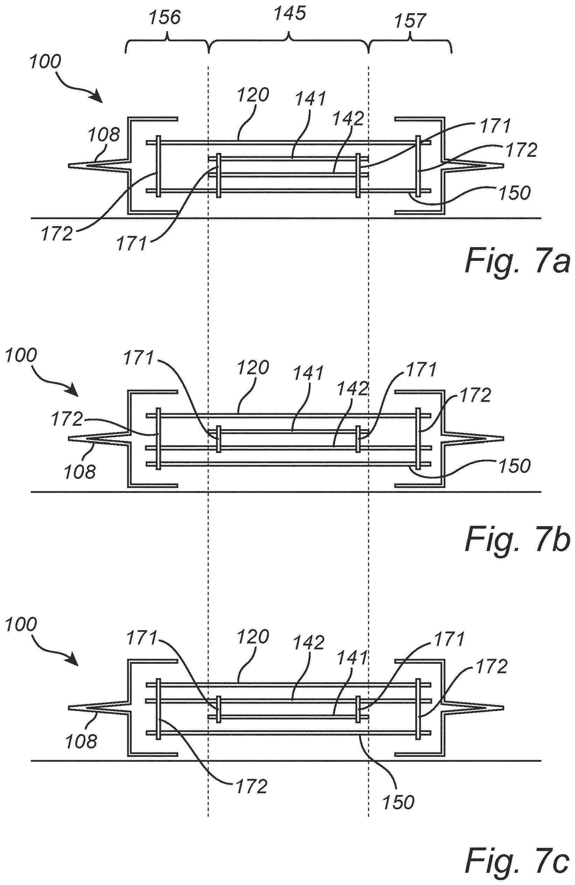

Figs. 7a-7c show cross sectional views of the absorbent assemblies in Figs. 6a-6c, with the position of constructional seams indicated. The layers 120, 141, 142, 150 in the absorbent assembly 100 of absorbent male underwear 1 as disclosed herein are preferably connected with each other by a first construction seam 171 and at least one second construction seam 172. Each construction seam connects a number of layers which is less than the total number of layers in the absorbent assembly 100. At least one layer in the first construction seam is different from a layer in the second construction seam, the absorbent assembly 100 being formed into a coherent component by at least one layer in the first construction seam 171 being the same as a layer in the second construction seam 172. Accordingly, no construction seam 171, 172 involves all layers of the absorbent assembly 100 but shares at least one layer with another construction seam 171, 172.

In Fig. 7a, the first layer 141 of textile absorbent material is attached to the second layer 142 of textile absorbent material and to the liquid impermeable layer by a first construction seam 171. The liquid permeable layer 120 is attached to the liquid impermeable layer 150 second construction seam 172. As can be seen in Fig. 7a the first and second construction seams are non-overlapping seams 171, 172 and are placed at a distance from each other in the x-y plane.

In Fig. 7b, the first layer 141 of textile absorbent material is attached to the second layer 142 of textile absorbent material by a first construction seam 171. The second layer 142 of textile absorbent material is attached to the liquid permeable layer 120 and to the liquid impermeable layer 150 by a second construction seam 172, the first and second construction seams 171, 172 being non-overlapping seams and being placed at a distance from each other in the x-y plane.

In Fig. 7c, the first layer 141 of textile absorbent material is attached to the second layer 142 of textile absorbent material by a first construction seam 171. The first layer 141 of textile absorbent material is attached to the liquid permeable layer 120 and to the liquid impermeable layer 150 by a second construction seam 172, the first and second construction seams 171, 172 being non-overlapping seams and being placed at a distance from each other in the x-y plane.

In all the arrangements shown in Figs. 7a-7c, every layer in the absorbent assembly 100 is directly connected to at least one other layer in the absorbent assembly 100 and is directly or indirectly connected to all other layers in the absorbent assembly 100.

The construction seams 171, 172 may be made by a flat stitch such as a flatlock stitch. The flatlock stitch may be a flatlock stitch in class 600 according to the international standard ISO 4915, such as a flatlock stitch type 607 according to the international standard ISO 4915.

Flatlock seams are formed from groups of threads; needle-, cover-, and looper threads, to create strong, band-shaped and flat seams. The needle threads extend back and forth through the front and back sides of the layer or layers of fabric, while the cover thread extends back and forth across the width of the seam only on the front side, and the looper thread extends back and forth across the width of the seam only on the back side of the fabric. Flatlock seams are advantageously used for next-to-the-skin garments such as e.g., underwear since these garments must be extra comfortable, so they don't chafe the skin or cause any other discomfort. Absorbent material such as e.g., the spacer material 138 described herein typically benefit from being joined by flatlock seams because a flatlock seam generally causes less compaction of the material than conventional seams which means that the flatlock seams are softer and more flexible than conventional seams and that an internal void structure of the material may be retained to a higher degree than when using conventional seams.

## Claims

1. Washable absorbent male underwear (1) comprising a textile pant (10) and an absorbent assembly (100), the textile pant (10) having an extension in a longitudinal direction (y), in a transverse direction (x) and in a thickness direction (z) perpendicular to the longitudinal direction (y), and the transverse direction (x) and comprising a front portion (11), a rear portion (16) and a crotch portion (15), the absorbent assembly (100) being permanently secured in the textile pant (10), the absorbent assembly (100) comprising
- a liquid permeable layer (120),
- a liquid impermeable layer (150); and
an absorbent structure (140) being arranged in the front portion (11) of the textile pant (10) between the liquid permeable layer (120) and the liquid impermeable layer (150) and comprising a first layer (141) of textile absorbent material, the first layer (141) of textile absorbent material having a first absorbent area; wherein
the first absorbent area defines an area of a first absorbent zone (145) of the absorbent assembly (100), the absorbent assembly (100) further comprising a leak protection zone (155) having an area which is defined by the area of the liquid impermeable layer (150), the absorbent structure (140) completely overlapping the leak protection zone (155), the area of the leak protection zone (155) being greater than the area of the first absorbent zone (145).

2. Washable absorbent male underwear according to claim 1, wherein the area of the first absorbent zone (145) is at most 70% of the area of the leak protection zone (155).

3. Washable absorbent male underwear according to claim 1 or 2, wherein the leak protection zone (155) extends in the transverse direction (x) laterally outside of the first absorbent zone (145).

4. Washable absorbent male underwear according to claim 3, wherein a portion (156, 157) of the leak protection zone (155) is arranged at each lateral side of the first absorbent zone (145) in a waist region of the front portion (11) of the absorbent male underwear (1).

5. Washable absorbent male underwear according to any one of the preceding claims, wherein the leak protection zone (155) extends in the longitudinal direction (y) outside of the first absorbent zone (145) and into the crotch portion of the absorbent male underwear (1).

6. Washable absorbent male underwear according to claim 5, wherein the leak protection zone (155) extends in the longitudinal direction (y) outside of the first absorbent zone (145) and through the crotch portion of the textile pant (10) and into the rear portion of the textile pant (10).

7. Washable absorbent male underwear according to any one of the preceding claims, wherein the leak protection zone (155) covers at most 70% of the area of the textile pant (10), such as at most 50% of the area of the textile pant (10) or at most 40% of the area of the textile pant (10).

8. Washable absorbent male underwear according to any one of the preceding claims, wherein the liquid impermeable layer (150) is a breathable layer.

9. Washable absorbent male underwear according to any one of the preceding claims, wherein the first absorbent layer (141) is a spacer fabric (138) layer comprising a top web (138a) and a bottom web (138b) which are interconnected by pile filaments (138c) extending in the thickness direction (z) between the top web (138a) and the bottom web (138b).

10. Washable absorbent male underwear according to any one of the preceding claims, wherein the absorbent assembly (100) is cup-shaped, at least within the first absorbent zone (145).

11. Washable absorbent male underwear according to claim 10, wherein the absorbent assembly (100) comprises first and second side-panels joined along long curved side-edges by a central join extending in the longitudinal direction (y) from a top edge to a bottom edge of the absorbent assembly (100), the central join extending perpendicular to the transverse direction (x).

12. Washable absorbent male underwear according to any one of the preceding claims, wherein the absorbent structure comprises a second layer (142) of textile absorbent material, the first layer of textile absorbent material completely overlapping with the second layer (142) of textile absorbent material.

13. Washable absorbent male underwear according to claim 12, wherein one of the first layer (141) of textile absorbent material and the second layer (142) of textile absorbent material is a liquid acquisition layer, such as a layer of spacer fabric material and the other of the first layer (141) of textile absorbent material and the second layer (142) of textile absorbent material is a liquid storage layer.

14. Washable absorbent male underwear according to claim 13, wherein the first layer (141) of textile absorbent material is the liquid acquisition layer and the second layer (142) of textile absorbent material is the liquid storage layer.

15. Washable absorbent male underwear according to claim 14, wherein one or more second absorbent zones (156', 157', 158') are formed by portions of the second layer (142) of textile absorbent material extending beyond a periphery (148) of the first absorbent zone (145).

16. Washable absorbent male underwear according to any one of claims 13 to 15, wherein the second textile absorbent layer is a liquid retaining layer arranged between the first layer (141) of textile absorbent material and the liquid impermeable layer (150).

17. Washable absorbent male underwear according to any one of claims 12 to 16, wherein the second layer (142) of textile absorbent material is thinner than the first layer (141) of textile absorbent material.

18. Washable absorbent male underwear according to any one of claims 12 to 17, wherein a second absorbent zone (158') is arranged in the crotch portion (15) of the textile pant (10).

19. Washable absorbent male underwear according to any one of claims 12 to 18, wherein a second absorbent zone (156', 157') is arranged at each lateral side of the first absorbent zone (145) in the front portion of the textile pant (10).

20. Washable absorbent male underwear according to any one of the preceding claims, wherein the layers in the absorbent assembly (100) are connected with each other by a first construction seam (171) and at least one second construction seam (172), each construction seam (171, 172) connecting a number of layers being less than a total number of layers in the absorbent assembly (100), a layer in the first construction seam (171) being the same as a layer (172) in the at least one second construction seam (172) and a layer in the first construction seam (171) being different from a layer in the at least one second construction seam (172).

21. Washable absorbent male underwear according to claim 20, wherein the first layer (141) of textile absorbent material is attached to the second layer (142) of textile absorbent material by the first construction seam (171) and the second layer (142) of textile absorbent material is attached to the liquid permeable layer (120) and to the liquid impermeable layer (150) by the second construction seam (172).

22. Washable absorbent male underwear according to claim 20, wherein the first layer (141) of textile absorbent material is attached to the second layer (142) of textile absorbent material and to the liquid impermeable layer (150) by the first construction seam (171) and the liquid permeable layer (120) is attached to the liquid impermeable layer (150) by the second construction seam (172).

23. Washable absorbent male underwear according to claim 20, 21 or 22, wherein at least the first construction seam (171) is a flat stitch, e.g. a flatlock stitch, such as a flatlock stitch in class 600 according to the international standard ISO 4915, such as a flatlock stitch type 607 according to the international standard ISO 4915.
